# EUROPEAN PATENT APPLICATION

(11) **EP 2 929 889 A1**
(43) Date of publication of application: **14.10.2015**
(21) Application number: 13860030.9
(22) Date of filing: 02.12.2013
(51) Int. Cl.: A61K 38/00, A61P 25/28, A61P 39/06

(54) **USE OF YAM GLYCOPROTEIN FOR IMPROVING ACTIVITY OF MITOCHONDRIAL OXIDATIVE METABOLIC ENZYME OF BRAIN CELL**

(30) Priority: 05.12.2012 CN 201210513861
(71) Applicant: Mamute, Duolikun, Xinjiang 830002 (CN)
(72) Inventor: Mamute, Duolikun, Xinjiang 830002 (CN)
(74) Representative: Cabinet Chaillot
(86) International application number: PCT/CN2013/088339
(87) International publication number: WO 2014/086258

(57) **Abstract**

A method for preparing for glycoprotein in Chinese yam to enhance the activity of metabolic enzymes of brain mitochondria may include steps of: grinding the Chinese yam into fine powers; adding water into the fine powers and heating up to 50-100°C to form a mixture, and the amount of water is 2 to 5 times as much as the Chinese yam; extracting the mixture twice, and combining the two extracting solutions; concentrating the extracting solution and adding 95% ethyl alcohol to obtain a first mixed liquid with 60-75% ethyl alcohol concentration and white sediments start to appear; separating the white sediments from a first supernate; adding 95-98% ethyl alcohol in the first supernate to obtain a second mixed liquid with 90-95% ethyl alcohol concentration and brown emplastic sediments start to appear; removing a second supernate; and collecting and drying the brown sediments to obtain brown glycoprotein in Chinese yam.

## Description

### FIELD OF THE INVENTION

The present invention relates to a glycoprotein in Chinese yam to enhance the activity of metabolic enzymes of brain mitochondria, and more particularly to enhance the activity of pyruvate dehydrogenase and *α-*ketoglutarate dehydrogenase in brain mitochondria.

### BACKGROUND OF THE INVENTION

Brain mitochondria is one of major organelles for maintaining normal physiological activity as well as the switch for controlling the apoptosis of brain cells. Under normal conditions, the major metabolizing enzymes pyruvate dehydrogenase and α-ketoglutarate dehydrogenase in mitochondria provide ATP generated from energy-yielding nutrient after oxidative metabolism to cells for normal physiological activity. However, if oxidative metabolism efficiency is reduced due to degenerative change of brain cells, or cerebral ischemia or anoxia arises resulted from cerebral infarction, and brain cells are not able to generate sufficient ATP to maintain normal physiological activity, a series of pathologic changes will occur, such as senile dementia, vascular dementia, stroke or other diseases. In other words, under the premise of maintaining basic blood and oxygen supply to brain cells, if the activity of major metabolizing enzymes in brain mitochondria can be increased, degenerated or damaged brain cells can be protected and restored to a certain degree in spite of cerebral ischemia or anoxia, and consequently these diseases arising from pathological changes can be effectively treated or controlled.

Glycoprotein in Chinese yam is a natural active ingredient contained in root and stems of Dioscorea plant, which is proved to effectively treat sleep apnea syndrome and resist oxidation and fatigue. In the present invention, a further research is conducted to show that glycoprotein from Chinese yam can remarkably elevate the activity of pyruvate dehydrogenase and α-ketoglutarate dehydrogenase in brain mitochondria and thus can treat and recover brain damage or disorder resulting from the reduction of oxidative metabolism in brain mitochondria caused by ischemia or anoxia or other factors.

### SUMMARY OF THE INVENTION

It is an object of the present invention to prove the function of glycoprotein from Chinese yam in the promotion of the activity of metabolizing enzymes in brain mitochondria. Through the animal experiments, identification and verification, it is demonstrated that the glycoprotein in Chinese yam can remarkably improve the activity of key metabolizing enzymes, pyruvate dehydrogenase and α-ketoglutarate dehydrogenase, in brain mitochondria, and therefore senile dementia, vascular dementia, cerebral infarction, cerebral palsy, brain cells degeneration and damage resulting from the lower activity of these metabolic enzymes can be treated effectively.

It is another object of the present invention to show that the glycoprotein in Chinese yam can promote the activity of metabolizing enzymes in brain mitochondria and increase the activity of pyruvate dehydrogenase and α-ketoglutarate dehydrogenase in brain mitochondria. It comes from dried root and stems of Dioscorea plant of the Dioscoreaceae or plant of other genera of the Dioscoreaceaea and it can be prepared as the following steps:

a. After grinding the root and stems of dried Chinese yam into fine power, adding water that is 2-5 times of the fine power and heating to 50-100°C, then extracting the liquid twice with the duration of 2-3 hours each time, and combining the liquid extracted twice after filtering and conducting vacuum concentration;

b. Adding 95% ethyl alcohol into the concentrated liquid obtained in step a to obtain a mixed liquid with 60-75% ethyl alcohol, and white sediments start to appear; leaving the mixed liquid for 3-6 hours and separating the white sediments with the supernate;

c. Putting 95-98% ethyl alcohol into the supernate from step b to obtain a mixed liquid with 90-95% ethyl alcohol, and brown emplastic sediments start to appear; leaving the mixed liquid for another 3-6 hours and removing the supernate away, and collecting and drying the brown sediments to obtain brown or dark brown crystalloid or emplastic solid Chinese yam glycoprotein.

It is noted that the glycoprotein from Chinese yam is used as active ingredient, and can be incorporated with medical carriers and subsidiary components.

In one embodiment, the glycoprotein can be made or included in capsules, granules, tablets, powder, oral liquid or syrup.

As stated above, the Chinese yam glycoprotein can greatly improve the activity of pyruvate dehydrogenase and α- ketoglutarate dehydrogenase in brain cell mitochondrion and cure acute or chronic brain damages caused by reduced oxidation efficiency of brain cell mitochondrion and diseases of brain function degradation caused by reduced brain cell oxidative metabolism. More importantly, it originates from natural plants and is easy to be extracted and prepared, which can be made into capsules, tablets, granules, oral liquid or other types.

### DETAILED DESCRIPTION OF THE INVENTION

The detailed description set forth below is intended as a description of the presently exemplary device provided in accordance with aspects of the present invention and is not intended to represent the only forms in which the present invention may be prepared or utilized. It is to be understood, rather, that the same or equivalent functions and components may be accomplished by different embodiments that are also intended to be encompassed within the spirit and scope of the invention.

Unless defined otherwise, all technical and scientific terms used herein have the same meaning as commonly understood to one of ordinary skill in the art to which this invention belongs. Although any methods, devices and materials similar or equivalent to those described can be used in the practice or testing of the invention, the exemplary methods, devices and materials are now described.

All publications mentioned are incorporated by reference for the purpose of describing and disclosing, for example, the designs and methodologies that are described in the publications that might be used in connection with the presently described invention. The publications listed or discussed above, below and throughout the text are provided solely for their disclosure prior to the filing date of the present application. Nothing herein is to be construed as an admission that the inventors are not entitled to antedate such disclosure by virtue of prior invention.

### EXAMPLE 1

In the first example, the glycoprotein is prepared by the following steps: (1) grinding 1000g natural Chinese yam into fine powers; (2) adding water that is twice as much into the fine powers and heating up to 50°C to form a mixture; (3) extracting the mixture twice, each extraction lasting for 2 hours, and combining the two extracting solutions together after filtration; (4) concentrating the extracting solution into 50% and adding 95% ethyl alcohol to obtain a first mixed liquid with 60% ethyl alcohol concentration and white sediments start to appear; (5) leaving the first mixed liquid for 3-6 hours and separating the sediments from a first supernate; (6) adding 95% ethyl alcohol in the first supernate to obtain a second mixed liquid with 90% ethyl alcohol concentration and brown sediments start to appear; (7) leaving the second mixed liquid for 3-6 hours and removing a second supernate; and (8) collecting and drying the brown sediments to obtain brown or dark brown emplastic solid crude Chinese yam glycoprotein.

### EXAMPLE 2

In Example 2, the glycoprotein is prepared by the following steps: (1) grinding 1000g natural Chinese yam into fine powers; (2) adding water that is three times as much into the fine powers and heating up to 70°C to form a mixture; (3) extracting the mixture twice, each extraction lasting for 3 hours, and combining the two extracting solutions together after filtration; (4) concentrating the extracting solution into 50% and adding 95% ethyl alcohol to obtain a first mixed liquid with 70% ethyl alcohol concentration and white sediments start to appear; (5) leaving the first mixed liquid for 3-6 hours and separating the sediments from a first supernate; (6) adding 95% ethyl alcohol in the first supernate to obtain a second mixed liquid with 90% ethyl alcohol concentration and brown sediments start to appear; (7) removing a second supernate; and (8) collecting and vacuum drying the brown sediments to obtain brown or dark brown emplastic solid crude Chinese yam glycoprotein.

### EXAMPLE 3

In Example 3, the glycoprotein is prepared by the following steps: (1) grinding 1000g natural Chinese yam into large particles; (2) adding water that is five times as much into the fine powers and heating up to 100°C to form a mixture; (3) extracting the mixture three times, each extraction lasting for 2 hours, and combining the three extracting solutions together after filtration; (4) concentrating the extracting solution into 50% and adding 95% ethyl alcohol to obtain a first mixed liquid with 75% ethyl alcohol concentration and white sediments start to appear; (5) leaving the first mixed liquid for 3-6 hours and separating the sediments from a first supernate; (6) adding 98% ethyl alcohol in the first supernate to obtain a second mixed liquid with 95% ethyl alcohol concentration and yellowish-brown emplastic sediments start to appear; (7) removing a second supernate; and (8) collecting and vacuum-freeze drying the sediments to obtain brown crystallized Chinese yam glycoprotein.

### EXAMPLE 4

In Example 4, a research on the effects of Chinese yam glycoprotein to the activity of pyruvate dehydrogenase and α- ketoglutarate dehydrogenase in brain cell mitochondrion of mice is conducted. The materials include enzyme-linked immunoassay kits of α- ketoglutarate dehydrogenase and pyruvate dehydrogenase of mice bought from Beijing Yonghui Biotechnology Co., Ltd. The animals are Kunming mice, half male and half female, weight (20±2) g, provided by Xinjiang Medical University Laboratory Animal Center. In the experiment, 27 mice are divided into 3 groups randomly, which is a control group, a small dose group of Chinese yam glycoprotein and a large dose group.

The Chinese yam glycoprotein is dissolved into pure water to prepare a solution of 0.42g/mL. In the large does group, the Chinese yam glycoprotein is given once per day by gavage in 0.42g/Kg (according the weight of the mouse) for large dose group, while 0.21g/Kg is for small dose group, and pure water is given for the control group. The experiment lasts for 10 consecutive days and the mice were killed one hour after the gavage on the tenth day. Their brain tissues were weighed and a certain amount of PBS (pH7.4) is added to fully mix with the brain tissues. The mixture is centrifuged for 20 minutes (2000-3000 RPM) and the supernate is collected and divided into several portions, one for inspection, and others are frozen for later use.

Table 1 is the comparison of enzyme content among control group and glycoprotein groups (α- ketoglutarate dehydrogenase U/mg protein):

| | **Control group** | | **Small dose group** | | **Large dose group** |
|---|---|---|---|---|---|
| 1 | 25.21 | 1 | 32.45 | 1 | 25.48 |
| 2 | 22.17 | 2 | 24.56 | 2 | 35.47 |
| 3 | 16.45 | 3 | 25.13 | 3 | 26.16 |
| 4 | 29.57 | 4 | 35.24 | 4 | 39.25 |
| 5 | 22.59 | 5 | 26.35 | 5 | 32.47 |
| 6 | 26.75 | 6 | 24.14 | 6 | 34.27 |
| 7 | 29.23 | 7 | 31.24 | 7 | 21.48 |
| 8 | 18.17 | 8 | 21.14 | 8 | 28.37 |
| 9 | 19.67 | 9 | 23.54 | 9 | 29.48 |
| Average: | 23.31222 | | 27.08778 | | 30.27 |
| | 4.450023 | | 4.470548 | | 5.268932 |

As shown in Table 1, the amount of α- ketoglutarate dehydrogenase in the brain tissues of the mice in the large and small dose groups is more than that in the control group.

Table 2 is the comparison of enzyme content among control group and glycoprotein groups (pyruvate dehydrogenase U /mg protein):

| | **Control group** | | **Small dose group** | | **Large dose group** |
|---|---|---|---|---|---|
| 1 | 15.26 | 1 | 19.57 | 1 | 14.21 |
| 2 | 12.56 | 2 | 15.68 | 2 | 20.47 |
| 3 | 16.24 | 3 | 15.48 | 3 | 16.89 |
| 4 | 9.23 | 4 | 14.21 | 4 | 19.54 |
| 5 | 12.58 | 5 | 11.24 | 5 | 12.47 |
| 6 | 16.54 | 6 | 10.47 | 6 | 14.63 |
| 7 | 19.47 | 7 | 16.57 | 7 | 11.24 |
| 8 | 11.14 | 8 | 21.47 | 8 | 18.54 |
| 9 | 12.47 | 9 | 16.32 | 9 | 19.47 |
| Average: | 13.94333 | | 15.66778 | | 16.38444 |
| | 3.168442 | | 3.516304 | | 3.366497 |

Likewise, as shown in Table 2, the amount of pyruvate dehydrogenase in the brain tissues of the mice in the large and small dose groups is more than that in the control group.

As shown in the Tables above, we can see that the glycoprotein in Chinese yam can greatly improve the activity of pyruvate dehydrogenase and *α-*ketoglutarate dehydrogenase in brain cell mitochondrion, which can be used to cure acute or chronic brain damages caused by reduced oxidation efficiency of brain cell mitochondrion and diseases of brain function degradation caused by reduced brain cell oxidative metabolism.

Having described the invention by the description and illustrations above, it should be understood that these are exemplary of the invention and are not to be considered as limiting. Accordingly, the invention is not to be considered as limited by the foregoing description, but includes any equivalents.

## Claims

1. A method for preparing for glycoprotein in Chinese yam to enhance the activity of pyruvate dehydrogenase and α-ketoglutarate dehydrogenase in brain mitochondria comprising steps of:
grinding a predetermined amount of Chinese yam into fine powers;
adding water into the fine powers and heating up to 50-100°C to form a mixture, wherein the amount of water is 2 to 5 times as much as the amount of the Chinese yam;
extracting the mixture twice, each extraction lasting for 2 to 3 hours, and combining the two extracting solutions together after filtration;
concentrating the extracting solution and adding 95% ethyl alcohol to obtain a first mixed liquid with 60-75% ethyl alcohol concentration and white sediments start to appear;
leaving the first mixed liquid for 3-6 hours and separating the white sediments from a first supernate;
adding 95-98% ethyl alcohol in the first supernate to obtain a second mixed liquid with 90-95% ethyl alcohol concentration and brown emplastic sediments start to appear;
leaving the second mixed liquid for 3-6 hours and removing a second supernate; and
collecting and drying the brown sediments to obtain brown or dark brown crystallized or emplastic solid glycoprotein in Chinese yam.
